(19) Europäisches Patentamt
European Patent Office
Office européen des brevets

(11) Veröffentlichungsnummer: **0 102 055**
**B1**

(12) **EUROPÄISCHE PATENTSCHRIFT**

(45) Veröffentlichungstag der Patentschrift: **11.05.88**

(51) Int. Cl.⁴: **C 07 D 277/82**

(21) Anmeldenummer: **83108316.7**

(22) Anmeldetag: **24.08.83**

(54) **Verfahren zur Herstellung halogensubstituierter 2-Aminobenzthiazole.**

(30) Priorität: **27.08.82 DE 3231885**

(43) Veröffentlichungstag der Anmeldung: **07.03.84 Patentblatt 84/10**

(45) Bekanntmachung des Hinweises auf die Patenterteilung: **11.05.88 Patentblatt 88/19**

(84) Benannte Vertragsstaaten: **BE CH DE FR GB IT LI**

(56) Entgegenhaltungen:
**EP - A - 0 003 141**
**DE - C - 537 105**
**FR - A - 2 298 548**
**US - A - 2 033 949**

(73) Patentinhaber: **HOECHST AKTIENGESELLSCHAFT, Postfach 80 03 20, D-6230 Frankfurt am Main 80 (DE)**

(72) Erfinder: **Rentél, Heinz, Dr., Höhenstrasse 45, D-6242 Kronberg/Taunus (DE)**
Erfinder: **Papenfuhs, Theodor, Dr., Heinrich-Bleicher-Strasse 40, D-6000 Frankfurt am Main 50 (DE)**

**EP 0 102 055 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Halogensubstituierte 2-Aminobenzthiazole sind wichtige Zwischenprodukte zur Herstellung von Farbstoffen (vgl. beispielsweise DE-AS 1 907 606, US-PS 3 502 645) und Vorprodukte für biologisch aktive Substanzen, z.B. von im Benzolkern halogensubstituierten 2-Chlorbenzthiazolen [vgl. beispielsweise CS-PS 164 118 (C.A. 87 (1977) 53258t), GB-PS 966 496].

Zur Synthese von 2-Aminobenzthiazolen sind im wesentlichen 2 Alternativen bekannt geworden:

1) Die alkalische Cyclisierung von o-Rhodananilinen [vgl. beispielsweise DE-PS 491 223 (Friedl. 16 2566/68), J. Am. Chem. Soc. 58 (1936) 1364/66, FR-PS 1 502 178]. Dieser Syntheseweg erfordert eine technisch und vor allem ökologisch problematische zumindest intermediäre Herstellung von Dirhodan (aus Alkalirhodanid und Halogen), liefert in den seltensten Fällen saubere Produkte und erfordert hohen Aufwand für die Abgas-(HSCN) und Abwasser-(Bromide)-Entsorgung.

2) Die oxidative Cyclisierung der entsprechenden Arylthioharnstoffe. Als oxidative Cyclisierungsmittel wurden Halogene (Chlor, Brom) und halogenabspaltende Verbindungen, vor allem Schwefelhalogenverbindungen, beispielsweise Dischwefeldichlorid, Thionylchlorid, Sulfurylchlorid vorgeschlagen (vgl. z.B. R.C. ELDERFIELD «Heterocyclic Compounds» Vol. 5, New York-London 1957, S. 581/82). In der EP-A 0 003 141 ist die Cyclisierung von Arylthioharnstoffen zu 2-Amino-arylenothiazolen, z.B. halogensubstituierten 2-Amino-benzthiazolen, mittels Thionylchlorid beschrieben. Bei diesen Synthesen fallen jedoch ansehnliche Mengen von elementarem Schwefel an, deren Entfernung einigen Aufwand erfordert.

Dieser Nachteil entfällt bei der Verwendung von Sulfurylchlorid. In der US-PS 2 033 949 ist die Synthese von fluorsubstituierten 2-Aminobenzthiazolen aus den entsprechenden Phenylthioharnstoffen durch Ringschlussreaktion mittels Sulfurylchlorid in einem geeigneten inerten Lösungsmittel, beispielsweise Chlorbenzol, beschrieben. Die Anwendung der beschriebenen Reaktionsbedingungen zur Synthese der analogen Chlor- und Bromverbindungen führt zu völlig unbefriedigenden niedrigen Ausbeuten.

Der Erfindung lag daher die Aufgabe zu Grunde, ein Verfahren zur Herstellung von chlor- bzw. bromsubstituierten 2-Aminobenzthiazolen zu finden, bei dem diese in hoher Ausbeute und Reinheit erhalten werden. Es wurde gefunden, dass dies erreicht wird, wenn man die Ringschlussreaktion mittels Sulfurylchlorid in einem inerten Lösungsmittel in Gegenwart eines Alkali- oder Erdalkali-oxids, -hydroxids oder -carbonats durchführt.

Gefunden wurde ein Verfahren zur Herstellung halogensubstituierter 2-Aminobenzthiazole der allgemeinen Formel (1)

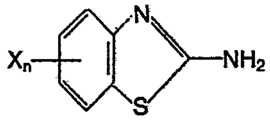

(1)

ausgehend von halogensubstituierten Phenylthioharnstoffen der Formel (2)

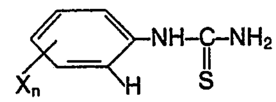

(2)

durch Ringschlussreaktion mittels Sulfurylchlorid in einem inerten Lösungsmittel, dadurch gekennzeichnet, dass X Chlor und/oder Brom und n eine ganze Zahl von 1 bis 4 bedeuten, und dass die Ringschlussreaktion in Gegenwart eines Alkali- oder Erdalkali-oxids, -hydroxids oder -carbonats erfolgt.

In der Regel stören geringe Mengen Wasser die Reaktion nicht, in manchen Fällen hat ein geringer Wasserzusatz von 0,5–10 Gew.-%, bezogen auf den eingesetzten Phenylthioharnstoff, überraschenderweise sogar einen positiven Effekt auf Ausbeute und Qualität der so erhaltenen Halogen-2-aminobenzthiazole. Wassermengen bis 25 Gew.-% sind tolerierbar und haben keinen nachteiligen Einfluss auf die Cyclisierungsreaktion und ihre Geschwindigkeit. Noch grössere Wassermengen führen jedoch zur Reaktionsverzögerung und/oder zu erhöhtem Verbrauch an Sulfurylchlorid und sollten daher vermieden werden.

Die Reaktionstemperatur liegt zweckmässigerweise im Bereich von etwa 30 bis etwa 80 °C, vorzugsweise im Bereich von etwa 45 bis etwa 65 °C.

Als Lösungsmittel eignen sich solche, in denen sich Sulfurylchlorid löst, die aber nicht mit diesem reagieren. Bevorzugte Beispiele sind alkylsubstituierte Aromaten wie Toluol und die Xylole, Halogenaliphaten wie Ethylenchlorid, Trichlorethan und Trichlorethylen, insbesondere Halogenaromaten wie Chlorbenzol, Brombenzol, die Di- und Trichlorbenzole und Chlortoluole. Es können auch Gemische aus den genannten Lösungsmitteln eingesetzt werden.

Die Menge des verwendeten Lösungsmittels ist nicht kritisch, muss aber so gross gewählt werden, dass das Umsetzungsgemisch in jeder Phase der Reaktion gut rührbar bleibt, um das Ausgasen der gasförmigen Umsetzungsprodukte HCl und $SO_2$ zu gewährleisten. Diese werden vorteilhaft in zwei- oder mehrstufigen Gaswäschen mit Wasser und nachfolgend Alkalilaugen zu wiederverwertbaren Salzsäure- und Alkalihydrogensulfitlösungen umgesetzt.

Beispiele für erfindungsgemäss als säurebindende Mittel einzusetzende Alkali- oder Erdalkaliverbindungen sind vorzugsweise Alkali- und Erdalkalicarbonate wie Natrium-, Kalium-, Magnesium-, Calcium- und Bariumcarbonat, Erdalkalihydroxide wie Magnesium-, Calcium- und Bariumhydroxid, insbesondere Erdalkalioxide wie Magnesium-, Calcium- und Bariumoxid. Es können auch Gemische aus den genannten Alkali- und Erdalkaliverbindungen eingesetzt werden. Als ganz besonders günstig hat sich Magnesiumoxid erwiesen.

Das säurebindende Mittel wird in der Regel mit einem Äquivalent je Mol Arylthioharnstoff eingesetzt. Geringe Überschüsse sind nicht problematisch, hohe Überschüsse, beispielsweise 2 oder mehr Äquivalente, verlangsamen die Cyclisierung so sehr, dass in technisch interessanten Reaktionszeiten keine vollständige Umsetzung mehr möglich ist. Wird dagegen weniger als 1 Äquivalent Säurebinder verwendet, so treten unerwünschte Nebenprodukte, z.B. halogensubstituierte Phenylharnstoffe, elementarer Schwefel und/oder Halogenbenzthiazolyl-halogenphenylguanidine auf, die bei vollständiger Abwesenheit des Säurebinders in vielen Fällen zu Hauptprodukten oder sogar zu alleinigen Umsetzungsprodukten werden können.

Die als Ausgangsverbindungen einzusetzenden halogensubstituierten Phenylthioharnstoffe der Formel (2) sind durch Umsetzung der entsprechenden halogensubstituierten Arylaminsalze mit Rhodaniden leicht zugänglich, insbesondere nach dem Verfahren gemäss EP-A 0 005 276, da es die Thioharnstoffe in grobkristalliner, nur wenig Wasser bindender Form liefert, die in der Regel als Feuchtware in das erfindungsgemässe Verfahren eingesetzt werden können und daher eine kostenträchtige Trocknung der Ausgangsprodukte entfallen kann.

In vielen Fällen kann sogar auf eine Isolierung der halogensubstituierten Phenylthioharnstoffe ganz verzichtet werden, nämlich dann, wenn ihre Synthese in dem zur erfindungsgemässen Ringschlussreaktion geeigneten Lösungsmittel durch Umsetzung von beispielsweise halogeniertem Phenylamin-sulfat (in suti aus der freien Base und konz. Schwefelsäure im verwendeten Lösungsmittel erzeugt) mit Alkalirhodanid erfolgt. In diesem Falle der Eintopf-Reaktion wird die gebildete Suspension des halogenierten Phenylthioharnstoffs direkt in Gegenwart der erfindungsgemäss einzusetzenden Alkali- bzw. Erdalkaliverbindungen mit Sulfurylchlorid umgesetzt.

Die zur quantitativen Cyclisierung erforderliche Menge an Sulfurylchlorid beträgt mindestens 1 Mol $SO_2Cl_2$ je Mol Phenylthioharnstoff. In der Praxis hat sich die Anwendung eines Überschusses von 10–100%, vorzugsweise von 30–70%, als günstig erwiesen, d.h. 1,1–2 Mol, vorzugsweise 1,3–1,7 Mol $SO_2Cl_2$ je Mol Phenylthioharnstoff.

Das erfindungsgemässe Verfahren wird in der Weise durchgeführt, dass man eine gerührte Lösungsmittel-Suspension des zu cyclisierenden halogenierten Phenylthioharnstoffs mit dem säurebindenden Mittel und gegebenenfalls mit Wasser versetzt, auf die Reaktionstemperatur erwärmt und dann innerhalb 1 bis 5 Stunden nach Massgabe der freiwerdenden Reaktionswärme bzw. Gasmengen die benötigte Menge Sulfurylchlorid zutropfen lässt.

Zur Aufbereitung kann entweder das während der Cyclisierung ausgefallene halogenierte 2-Aminobenzthiazoliumsalz, z.B. das Chlorid und/oder Sulfat, durch Filtration vom Lösungsmittel getrennt und, gegebenenfalls nach Trocknung, durch Behandlung mit wässriger Alkalilauge in das freie halogenierte 2-Aminobenzthiazol überführt werden (bei einigen Verbindungen wegen eines beobachteten Reinigungseffektes vorteilhaft), oder das Umsetzungsgemisch kann, gegebenenfalls nach vorheriger Neutralisation mit Ammoniak oder anderen Alkalien, durch Wasserdampfdestillation vom Lösungsmittel befreit und das halogenierte 2-Aminobenzthiazol aus der resultierenden wässrigen Suspension, gegebenenfalls nach vorheriger Abkühlung, als Salz oder freie Base durch Filtration isoliert werden.

Nach dem erfindungsgemässen Verfahren lassen sich auch halogensubstituierte 2-Aminoarylenothiazole mit mehr als einem Benzolkern synthetisieren, z.B. halogensubstituierte 2-Aminonaphthylenothiazole der Formel (1a) bzw. (1b)

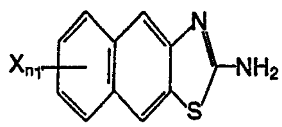

bzw.

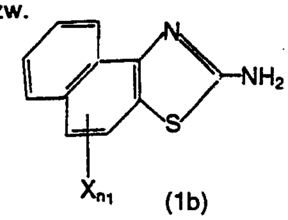

(1a) (1b)

ausgehend von halogensubstituierten Naphthylthioharnstoffen der Formel (2a) bzw. (2b)

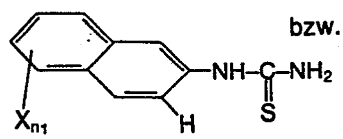

bzw.

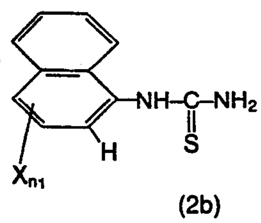

(2a) (2b)

$n_1$ hat darin die Bedeutung einer ganzen Zahl von 1 bis 6.

Die nachfolgenden Beispiele sollen das erfindungsgemässe Verfahren näher erläutern, ohne es zu beschränken. Teile und Prozentzahlen beziehen sich auf das Gewicht, falls nicht etwas anderes angegeben ist.

Beispiel 1

186,5 Teile 4-Chlorphenylthioharnstoff und 20 Teile Magnesiumoxid wurden unter Rühren in 800 Teile Chlorbenzol eingetragen und die Suspension auf etwa 50 °C geheizt. Bei dieser Temperatur liess man eine Mischung aus 222,7 Teilen Sulfurylchlorid und 150 Teilen Chlorbenzol im Laufe von etwa 2½ Stunden zutropfen. Anschliessend wurde etwa 3 Stunden bei 50 bis 55 °C nachgerührt bis die Gasentwicklung beendet war. Die entweichenden Gase (HCl und $SO_2$) wurden in einer 2-stufigen, mit Wasser bzw. Natronlauge betriebenen Absorptionsanlage absorbiert und dabei zu Salzsäure bzw. Natriumbisulfit umgesetzt. Nach erfolgtem Ringschluss liess man 200 Teile Wasser zutropfen und stellte mit etwa 200 Teilen 33%igem Ammoniak auf einen pH von 8–8,5. Nun wurde das Chlorbenzol mittels Wasserdampf abdestilliert, das 2-Amino-6-chlorbenzthiazol bei Raumtemperatur abgesaugt, mit Wasser gewaschen und im Umlufttrockenschrank bei etwa 80 °C getrocknet. Ausbeute: 171,3 Teile = 92,8% d. Th., Fp: 188–195 °C.

Beispiel 2

186,5 Teile 4-Chlorphenylthioharnstoff und 50 Teile Calziumcarbonat wurden unter Rühren in 900 Teile o-Dichlorbenzol eingetragen und die Suspension bei einer Temperatur von 45–50 °C innerhalb von 3 Stunden mit 223 Teilen Sulfurylchlorid versetzt. Man rührte anschliessend 3 Stunden bei 50–55 °C nach bis die Gasentwicklung beendet war. Schliesslich wurde über eine Glasfritte abgesaugt, zuerst mit Chlorbenzol und dann mit Petroläther (Siedebereich 80–100 °C) gewaschen und getrocknet.

Das erhaltene Rohprodukt wurde in 600 Teilen 96%iger Schwefelsäure verrührt. Die erhaltene Suspension liess man in 3000 Teile Wasser einlaufen, heizte auf etwa 90 °C auf, versetzte mit 30 Teilen Aktivkohle und filtrierte ab. Das geklärte Filtrat wurde mit 25%iger Natronlauge (auch konz. Ammoniak kann dazu verwendet werden) auf pH 10–11 gestellt, der Niederschlag bei Raumtemperatur abgesaugt, neutralgewaschen und getrocknet.

Ausbeute: 194 Teile 2-Amino-6-chlorbenzthiazol = 87,5% d. Th.

Beispiel 3

101,6 Teile 4-Chlorphenylthioharnstoff (91,7%ig feucht) und 35 Teile Bariumoxid wurden unter Rühren in 400 Teile Chlorbenzol eingetragen. Bei einer Temperatur von 52–55 °C liess man innerhalb von etwa 3 Stunden ein Gemisch von 116,9 Teilen Sulfurylchlorid und 70,0 Teilen Chlorbenzol zutropfen. Danach wurde etwa 5 Stunden bei dieser Temperatur nachgerührt bis die Gasentwicklung beendet war. Nach erfolgtem Ringschluss wurde die Aufarbeitung des Rohproduktes wie in Beispiel 2 beschrieben ausgeführt.

Ausbeute: 82 Teile 2-Amino-6-chlorbenzthiazol = 88,8% d. Th.

Beispiel 4

186,5 Teile 4-Chlorphenylthioharnstoff und 42 Teile Magnesiumcarbonat wurden unter Rühren in 1000 Teile Chlortoluol eingetragen und die Suspension auf etwa 50 °C geheizt. Bei dieser Temperatur liess man 225 Teile Sulfurylchlorid im Laufe von etwa 2½ Stunden zutropfen und anschliessend 3 Stunden nachrühren bis die Gasentwicklung beendet war. Danach wurde mit 200 Teilen Wasser versetzt und mit konz. Ammoniak auf einen pH von 8–8,5 gestellt. Nach erfolgter Entfernung des Chlortoluols mittels Wasserdampfdestillation wurde das 2-Amino-6-chlorbenzthiazol bei Raumtemperatur abgesaugt, mit Wasser gewaschen und getrocknet.

Ausbeute: 167 Teile = 90,5% d. Th.

Beispiel 5

Eine Mischung aus 221 Teilen 3,4-Dichlorphenylthioharnstoff, 1000 Teilen o-Dichlorbenzol und 25 Teilen Magnesiumoxid wurde unter Rühren bei 40–45 °C innerhalb von etwa 2 Stunden gleichmässig mit 230 Teilen Sulfurylchlorid versetzt. Nach Beendigung der Gasentwicklung wurde filtriert, der Filterrückstand in 500 Teile Wasser eingetragen und das anhaftende o-Dichlorbenzol mit Wasserdampf abgeblasen. Dann wurde mit etwa 85 Teilen 25%igem wässrigen Ammoniak auf pH 7–8 gestellt, abgekühlt und das ausgefallene Gemisch aus 2-Amino-5,6- bzw. 6,7-dichlorbenzthiazol durch Filtration isoliert. Nach dem Trocknen erhielt man 205 Teile Endprodukt mit einem Isomerenverhältnis von 6:4 und vom Schmelzpunkt 180–198 °C (93,7% d. Th.).

Beispiel 6

Es wurde wie im Beispiel 5 beschrieben vorgegangen mit dem Unterschied, dass man das Magnesiumoxid durch eine äquivalente Menge Kaliumcarbonat ersetzte. Man erhielt ein vergleichbares Ergebnis.

Beispiel 7

Zu einer gerührten Mischung aus 186,5 Teilen 2-Chlorphenylthioharnstoff, 500 Teilen Toluol, 300 Teilen Chlorbenzol und 11 Teilen Natriumcarbonat liess man bei 35–40 °C innerhalb von etwa 2 Stunden 210 Teile Sulfurylchlorid zutropfen. Nach beendeter Gasentwicklung wurden 250 Teile Wasser zugegeben, das Lösungsmittelgemisch mit Wasserdampf abdestilliert und die verbleibende wässrige Phase mit 500 Teilen 30%iger Salzsäure versetzt. Man filtrierte bei etwa 90 °C vom Ungelösten, kühlte das Filtrat auf etwa 20 °C ab und fällte aus dem als Hydrochlorid gelösten 2-Amino-4-chlorbenzthiazol das freie Amin durch Zugabe von überschüssiger Natronlauge bis zu einem End-pH von 8,5–9,0. Das Produkt wurde abgesaugt, mit Wasser neutral gewaschen und getrocknet.

Man erhielt 170 Teile 2-Amino-4-chlorbenzthiazol vom Schmelzpunkt 199–200 °C (92,4% d. Th.).

Beispiele 8–12

Entsprechend den Angaben der Beispiele 1–7 wurden gemäss dem Fliessschema

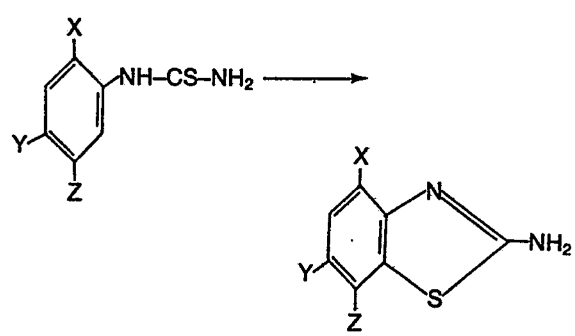

weitere Phenylthioharnstoffe zu 2-Aminobenzthiazolen cyclisiert. Die erhaltenen Verbindungen sind mit Substituentenstellung, Ausbeute und Schmelzpunkt in nachfolgender Tabelle zusammengefasst:

| Beispiel | X | Y | Z | Ausbeute | Schmelzpunkt |
|---|---|---|---|---|---|
| 8 | H | Br | H | 92,5% | 210–212°C |
| 9 | Cl | Cl | H | 94,0% | 248–251°C |
| 10 | Br | Br | H | 89,2% | 227–231°C |
| 11 | Cl | Cl | Cl | 87,9% | 292–294°C |
| 12 | Cl | H | Cl | 89,8% | 231–234°C |

Vergleichsbeispiel 1

186,5 Teile 4-Chlorphenylthioharnstoff wurden unter Rühren in 850 Teilen Chlorbenzol suspendiert und dann unter Rühren während etwa 3 Stunden bei 40–45 °C gleichmässig mit 185 Teilen Sulfurylchlorid versetzt. Nachdem die sofort einsetzende Gasentwicklung beendet war, wurde die entstandene, nur noch schwierig rührbare Suspension durch Einblasen von Wasserdampf destillativ vom Chlorbenzol befreit. Die verbleibende Suspension des 2-Amino-6-chlor-benzthiazoliumchlorids wurde mit 25%iger Ammoniaklösung auf pH 8 gestellt, wobei sich das rohe 2-Amino-6-chlorbenzthiazol zu einer klebrigen Masse zusammenballte. Durch Dekantieren und Trocknen erhielt man 146,3 Teile 2-Amino-6-chlor-benzthiazol mit einem Reingehalt von etwa 85% (= 124,4 Teile 100%ig, 67,4% d. Th.).

Eine Reinigung durch Umlösen oder Umkristallisieren war schwierig und sehr verlustreich. Im besten Falle wurden aus dem Rohprodukt durch mehrmaliges Umlösen über das Hydrochlorid mit adsorptiver Reinigung der wässrigen Lösung mit Aktivkohle 69,0 Teile 2-Amino-6-chlorbenzthiazol mit einem Reingehalt > 95% (37,4% d. Th.) erhalten.

Vergleichsbeispiel 2

231 Teile 4-Bromphenylthioharnstoff, suspendiert in 1200 Teilen o-Dichlorbenzol, wurden innerhalb 4 Stunden unter Rühren bei 45–50 °C gleichmässig mit 190 Teilen Sulfurylchlorid versetzt. Nach Beendigung der Gasentwicklung wurde auf etwa 20 °C abgekühlt, das ausgefallene rohe 2-Amino-6-brombenzthiazoliumchlorid abgesaugt und mit o-Dichlorbenzol gewaschen. Der lösungsmittelfeuchte Filterkuchen wurde in 1000 Teilen Wasser suspendiert, das anhaftende o-Dichlorbenzol durch Einblasen von Wasserdampf abdestilliert und die dabei erhaltene Lösung des 2-Amino-6-brombenzthiazoliumchlorids nach Zugabe von 10 Teilen Aktivkohle bei 95 °C vom schmierigen Ungelösten filtriert. Das klare Filtrat wurde mit 25%iger Natronlauge auf pH 8 gestellt, wobei das freie 2-Amino-6-brombenzthiazol als gelblicher, leicht klebriger Niederschlag ausfiel. Nach Abkühlen liess sich der dabei glasig erstarrte Feststoff durch Filtration isolieren und bei 40–50 °C im Vakuum trocknen. Man erhielt 167 Teile 2-Amino-6-brombenzthiazol mit einem Reingehalt von etwa 88% (= 147,1 Teile 100%ig, 64,2% d. Th.). Durch mehrfache Umlösung aus verdünnter Salzsäure unter Zusatz von Aktivkohle konnte daraus 2-Amino-6-brombenzthiazol mit einem Reingehalt > 95% erhalten werden. Diese Reinigung war aber sehr aufwendig und verlustreich und lieferte bestenfalls 82 Teile der gesuchten Verbindung (35,8% d. Th., bezogen auf eingesetzten 4-Bromphenylthioharnstoff).

**Patentansprüche**

1. Verfahren zur Herstellung halogensubstituierter 2-Aminobenzthiazole der allgemeinen Formel (1)

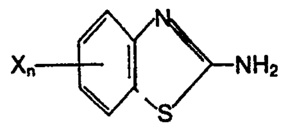

(1)

worin X Chlor und/oder Brom und n eine ganze Zahl von 1 bis 4 bedeuten,
ausgehend von halogensubstituierten Phenylthioharnstoffen der Formel (2)

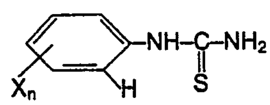

(2)

worin X und n die vorstehend genannten Bedeutungen haben, durch Ringschlussreaktion mittels Sulfurylchlorid in einem inerten Lösungsmittel, dadurch gekennzeichnet, dass die Ringschlussreaktion in Gegenwart eines Alkali- oder Erdalkalioxids, -hydroxids oder -carbonats erfolgt.

2. Verfahren nach Anspruch 1, dadurch gekennzeichnet, dass die Ringschlussreaktion bei einer Temperatur im Bereich von etwa 30 bis etwa 80 °C durchgeführt wird.

3. Verfahren nach Anspruch 2, dadurch gekennzeichnet, dass die Ringschlussreaktion bei einer Temperatur im Bereich von etwa 45 bis etwa 65 °C durchgeführt wird.

4. Verfahren nach einem der Ansprüche 1 bis 3, dadurch gekennzeichnet, dass die Ringschlussreaktion unter Zusatz von 0,5 bis 10 Gew.-% Wasser, bezogen auf den eingesetzten Phenylthioharnstoff, durchgeführt wird.

5. Verfahren nach einem der Ansprüche 1 bis 4, dadurch gekennzeichnet, dass 1,1 bis 2 Mol Sulfurylchlorid je Mol Phenylthioharnstoff eingesetzt werden.

6. Verfahren nach Anspruch 5, dadurch gekennzeichnet, dass 1,3 bis 1,7 Mol Sulfurylchlorid je Mol Phenylthioharnstoff eingesetzt werden.

7. Verfahren nach einem der Ansprüche 1 bis 6, dadurch gekennzeichnet, dass die Ringschlussreaktion in Gegenwart von Magnesiumoxid und/oder Calciumoxid und/oder Bariumoxid erfolgt.

8. Verfahren nach einem der Ansprüche 1 bis 7, dadurch gekennzeichnet, dass die Ringschlussreaktion in Gegenwart von Magnesiumoxid erfolgt.

9. Verfahren nach einem der Ansprüche 1 bis 8, dadurch gekennzeichnet, dass als Lösungsmittel alkylsubstituierte Aromaten, Halogenaliphaten, insbesondere Halogenaromaten eingesetzt werden.

10. Verfahren nach einem der Ansprüche 1 bis 9, dadurch gekennzeichnet, dass man ein Lösungsmittel oder ein Gemisch von Lösungsmitteln aus der Reihe Toluol, Xylole, Ethylenchlorid, Trichlorethan, Trichlorethylen einsetzt.

11. Verfahren nach einem der Ansprüche 1 bis 9, dadurch gekennzeichnet, dass man ein Lösungsmittel oder ein Gemisch von Lösungsmitteln aus der Reihe Chlorbenzol, Brombenzol, Dichlorbenzole, Trichlorbenzole, Chlortoluole einsetzt.

**Claims**

1. A process for the preparation of halogen-substituted 2-aminobenzothiazoles of the formula (1)

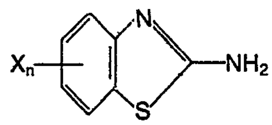 (1)

in which X is chlorine and/or bromine and n is an integer from 1 to 4, starting from halogen-substituted phenylthioureas of the formula (2)

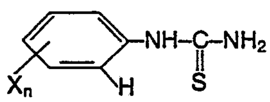 (2)

in which X and n have the abovementioned meanings, by cyclization reaction with sulfuryl chloride in an inert solvent, wherein the cyclization reaction is carried out in the presence of an alkali metal or alkaline earth metal oxide, hydroxide or carbonate.

2. The process as claimed in claim 1, wherein the cyclization reaction is carried out at a temperature in the range from about 30 to about 80 °C.

3. The process as claimed in claim 2, wherein the cyclization reaction is carried out at a temperature in the range from about 45 to about 65 °C.

4. The process as claimed in any of claims 1 to 3, wherein the cyclization reaction is carried out with addition of 0.5 to 10% by weight of water, relative to the phenylthiourea used.

5. The process as claimed in any of claims 1 to 4, wherein 1.1 to 2 mol of sulfuryl chloride per mol of phenylthiourea are used.

6. The process as claimed in claim 5, wherein 1.3 to 1.7 mol of sulfuryl chloride per mol of phenylthiourea are used.

7. The process as claimed in any of claims 1 to 6, wherein the cyclization reaction is carried out in the presence of magnesium oxide and/or calcium oxide and/or barium oxide.

8. The process as claimed in any of claims 1 to 7, wherein the cyclization reaction is carried out in the presence of magnesium oxide.

9. The process as claimed in any of claims 1 to 8, wherein alkyl-substituted aromatic substances, haloaliphatic substances, and especially haloaromatic substances are used as solvents.

10. The process as claimed in any of claims 1 to 9, wherein a solvent or a mixture of solvents selected from the group of toluene, xylenes, ethylene chloride, trichloroethane, trichloroethylene is used.

11. The process as claimed in any of claims 1 to 9, wherein a solvent or a mixture of solvents selected from the group of chlorobenzene, bromobenzene, dichlorobenzenes, trichlorobenzenes, chlorotoluenes is used.

**Revendications**

1. Procédé de préparation d'amino-2 benzothiazoles halogénés répondant à la formule générale (1):

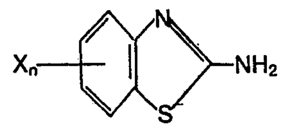 (1)

dans laquelle X représente le chlore et/ou le brome et n désigne un nombre entier de 1 à 4, à partir de phényl-thio-urées halogénées répondant à la formule (2):

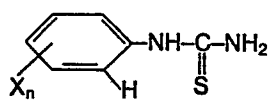 (2)

dans laquelle X et n ont les significations précédemment données, par cyclisation au moyen du chlorure de sulfuryle dans un solvant inerte, procédé caractérisé en ce qu'on effectue la réaction de cyclisation en présence d'un oxyde, d'un hydroxyde ou d'un carbonate de métal alcalin ou de métal alcalino-terreux.

2. Procédé selon la revendication 1, caractérisé en ce qu'on effectue la réaction de cyclisation à une température comprise entre environ 30 et environ 80 °C.

3. Procédé selon la revendication 2, caractérisé en ce qu'on effectue la réaction de cyclisation à une température comprise entre environ 45 et environ 65 °C.

4. Procédé selon l'une quelconque des revendications 1 à 3, caractérisé en ce qu'on effectue la réaction de cyclisation en présence de 0,5 à 10% en poids d'eau par rapport à la phényl-thio-urée mise en jeu.

5. Procédé selon l'une quelconque des revendications 1 à 4, caractérisé en ce qu'on utilise de 1,1 à 2 mol de chlorure de sulfuryle par mole de phényl-thio-urée.

6. Procédé selon la revendication 5, caractérisé en ce qu'on utilise de 1,3 à 1,7 mol de chlorure de sulfuryle par mole de phényl-thio-urée.

7. Procédé selon l'une quelconque des revendications 1 à 6, caractérisé en ce qu'on effectue la réaction de cyclisation en présence d'oxyde de magnésium et/ou d'oxyde de calcium et/ou d'oxyde de baryum.

8. Procédé selon l'une quelconque des revendications 1 à 7, caractérisé en ce qu'on effectue la réaction de cyclisation en présence d'oxyde de magnésium.

9. Procédé selon l'une quelconque des revendications 1 à 8, caractérisé en ce qu'on utilise,

comme solvants, des hydrocarbures aromatiques porteurs de radicaux alkyles, des hydrocarbures aliphatiques halogénés ou, plus spécialement, des hydrocarbures aromatiques halogénés.

10. Procédé selon l'une quelconque des revendications 1 à 9, caractérisé en ce qu'on utilise un solvant ou un mélange de solvants pris dans l'ensemble constitué par le toluène, les xylènes, le chlorure d'éthylène, le trichloréthane et le trichloréthylène.

11. Procédé selon l'une quelconque des revendications 1 à 9, caractérisé en ce qu'on utilise un solvant ou un mélange de solvants pris dans l'ensemble constitué par le chlorobenzène, le bromobenzène, les dichlorobenzènes, les trichlorobenzènes et les chlorotoluènes.